# EUROPEAN PATENT SPECIFICATION

(11) **EP 0 117 440 B1**
(45) Date of publication and mention of the grant of the patent: **07.04.1993**
(21) Application number: 84100836.0
(22) Date of filing: 26.01.1984
(51) Int. Cl.: C07H 21/00, G01N 33/58

(54) **Methods and structures employing non-radioactive chemically-labeled polynucleotide probes**
Verfahren und Strukturen in welchen chemisch markierte, nichtradioaktive Polynucleotidesonden benutzt werden
Méthodes et structures utilisant des sondes de polynucléotides marqués chimiquement et non-radioactifs

(30) Priority: 27.01.1983 US 461469
(43) Date of publication of application: 05.09.1984
(60) Divisional application: 92114727.8
(73) Proprietor: ENZO BIOCHEM, INC., New York, N.Y. 10013 (US)
(72) Inventor: Stavrianopoulos, Jannis G., New York New York 10019 (US); Kirtikar, Dollie, Elmhurst New York 11373 (US); Johnston, Kenneth H., New York New York 10014 (US); Thalenfeld, Barbara E., New York New York 10016 (US)
(74) Representative: VOSSIUS & PARTNER

(56) References cited:
- EP-A- 0 063 879
- EP-A- 0 097 373
- WO-A-83/02277
- US-A- 4 358 535
- Methods in Enzymology, Vol. XXXIV, Part B, pp. 59-72; M.B. Jacoby et al.
- Practical Guide for use in Affinity Chromatography IBF 1979, pp. 28-29

## Description

In the determination of the presence or the identity of genetic material, such as DNA genetic material, it has been proposed to denature the genetic material to form single-stranded DNA or single-stranded genetic material. The single-stranded genetic material is then fixed to a solid support and contacted with a probe, such as a DNA probe, having in its make up bases complementary to the make up of the fixed genetic material to be identified and/or determined. The contacting of the single-stranded genetic material along with the single-stranded probe is carried out under conditions to effect hybridization of the genetic material to be determined or identified and the probe.

Radioactively-labeled probes, such as radioactively-labeled single-stranded DNA probes, have been employed. U.S. Patent 4,358,535 discloses a method of identifying a pathogen present in the clinical sample by denaturing the genetic material present in the clinical sample to form single-stranded genetic material thereof and to fix the resulting single-stranded genetic material characterizing the pathogen to an inert support or surface. The method is especially suitable for screening of bacterial colonies for a specific polynucleotide sequence. The support has to be porous if it is placed on a nutrient medium in order to increase the cell number. The thus-fixed single-stranded genetic material characterizing or identifying the pathogen is brought into contact with a radioactive single-stranded probe under hybridizing conditions to effect duplex form or double-strand formation of the genetic material derived from the pathogen and the probe. The presence of the resulting formed duplex between the probe and the pathogen genetic material would then be detected by an insoluble signal and would confirm the presence and/or identity of the pathogen.

The disadvantages of employing a radioactively-labeled probe, such as a radioactively-labeled DNA probe, for the identification of genetic material are well known to those skilled in the art. Such disadvantages include not only the precautions and hazards involved in handling the radioactive material but also the short life of such radioactive material and expense in connection with the handling and use of such radioactively-labeled DNA probes.

It is known to chemically-label nucleotides and polynucleotides to avoid the hazards and/or difficulties associated when such compounds are radioactively-labeled. For example in the article by P.R. Langer, A.A. Waldrop and D.C. Ward entitled "Enzymatic Synthesis of Biotin-Labeled Polynucleotides: Novel Nucleic Acid Affinity Probes", in Proc. Natl. Acad. Sci., USA, Vol. 78, No. 11, pp. 6633-6637, November 1981, there are described analogs of dUTP and UTP that contain a biotin molecule bound to the C-5 position of the pyrimidine ring through an allylamine linker arm. The biotin-labeled nucleotides are efficient substrates for a variety of DNA and RNA polymerases in vitro. Polynucleotides containing low levels of biotin substitution (50 molecules or fewer per kilobase) have denaturation, reassociation and hybridization characteristics similar to those of unsubstituted controls. Biotin-labeled polynucleotides, both single and double stranded, are selectively and quantitatively retained on avidin-Sepharose, even after extensive washing with 8M urea, 6M guanidine hydrochloride or 99% formamide. In addition, biotin-labeled nucleotides can be selectively immunoprecipitated in the presence of antibiotin antibody and Staphylococcus aurea, Protein A. These unique features of biotin-labeled polynucleotides suggest that they are useful affinity probes for the detection and isolation of specific DNA and RNA sequences.

Compounds or nucleotides have also been prepared which can be incorporated into DNA, such as double-stranded DNA, and which are useful for the preparation of non-radioactive chemically-labeled DNA probes.

In EP-A- 0 063 879 the subject matter of the above-identified article is disclosed, and additionally there are disclosed compounds having the structure: 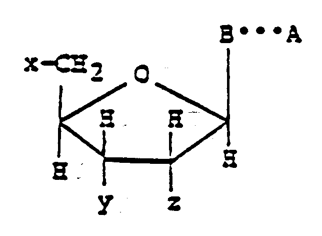
wherein B represents a purine, deazapurine, or pyrimidine moiety covalently bonded to the C^{1'}-position of the sugar moiety, provided that when B is purine or 7-deazapurine, it is attached at the N⁹-position of the purine or deazapurine, and when B is pyrimidine, it is attached at the N¹-position;
wherein A represents a moiety consisting of at least three carbon atoms which is capable of forming a detectable complex with a polypeptide when the compound is incorporated into a double-stranded ribonucleic acid, deoxyribonucleic acid duplex, or DNA-RNA hybrid;
wherein the dotted line represents a chemical linkage joining B and A, provided that if B is purine, the linkage is attached to the 9-position of the purine, if B is 7-deazapurine, the linkage is attached to the 7-position of the deazapurine, and if B is pyrimidine, the linkage is attached to the 5-position of the pyrimidine; and
wherein each of x, y and z represents 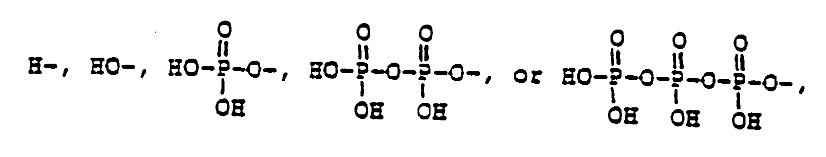
These compounds are widely useful as probes in biomedical research and recombinant DNA technology.

Particularly useful are compounds encompassed within this structure which additionally have one or more of the following characteristics: A is non-aromatic; A is at least C₅; the chemical linkage joining B and A includes an α-olefinic bond; A is biotin or iminobiotin; and B is a pyrimidine or 7-deazapurine.

EP-A-0 063 879 also discloses compounds having the structure: 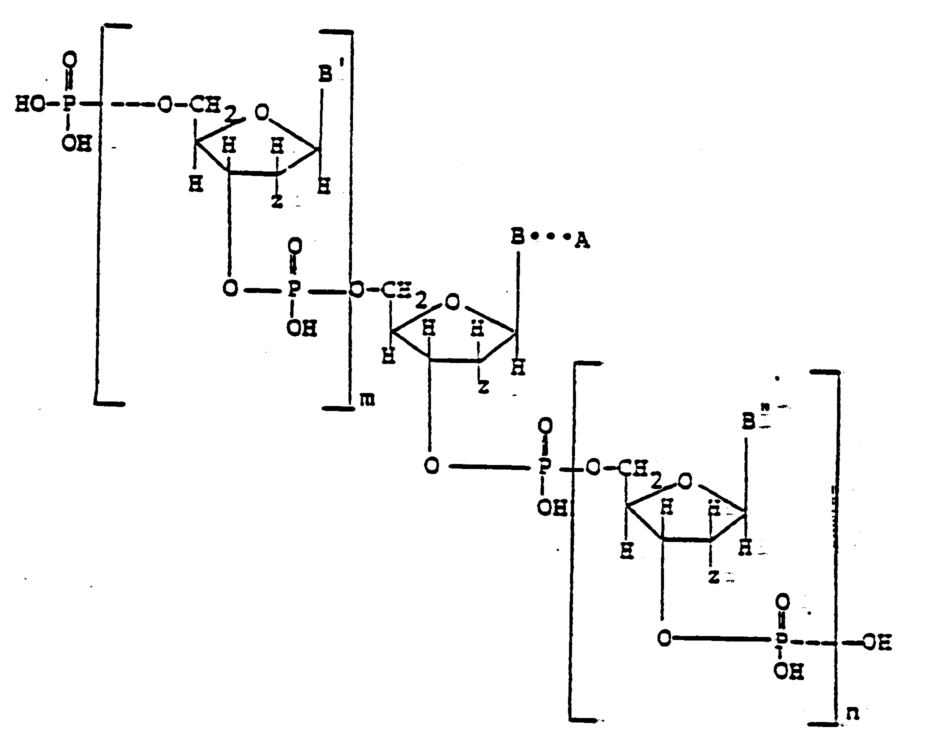
wherein each of B, B', and B'' represents a purine, 7-deazapurine, or pyrimidine moiety covalently bonded to the C^{1'}-position of the sugar moiety, provided that whenever B, B', or B'' is purine or 7-deazapurine, it is attached at the N⁹-position of the purine or 7-deazapurine, and whenever B, B', or B'' is pyrimidine, it is attached at the N¹-position;
wherein A represents a moiety consisting of at least three carbon atoms which is capable of forming a detectable complex with a polypeptide when the compound is incorporated into a double-stranded duplex formed with a complementary ribonucleic or deoxyribonucleic acid molecule;
wherein the dotted line represents a chemical linkage joining B and A, provided that if B is purine, the linkage is attached to the 8-position of the purine, if B is 7-deazapurine, the linkage is attached to the 8-position of the deazapurine, and if B is pyrimidine, the linkage is attached to the 5-position of the pyrimidine;
wherein z represents H- or HO-; and
wherein m and n represent integers from 0 up to about 100,000.

These compounds can be prepared by enzymatic polymerization of a mixture of nucleotides which include the modified nucleotides of this invention. Alternatively, nucleotides present in oligo- or polynucleotides may be modified using chemical methods.

The chemically-labeled or modified nucleotides described in the above-referred PNAS article and in EP-A-0 063 879 as indicated hereinabove have the structure: 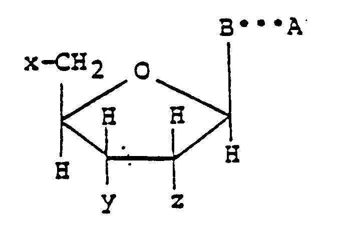
wherein B, A the dotted line and x,y and z are as defined above.

Although according to EP-A-0 063 879 in principal, all compounds encompassed within the above structural formula may be prepared and used, certain of the compounds are more readily prepared or used or both, and therefore are preferred.

Thus, although according to EP-A-0 063 879 purines, pyrimidines and 7-deazapurines are in principal useful, pyrimidines and 7-deazapurines are preferred since purine substitution at the 8-position tends to render the nucleotides ineffective as polymerase substrates. Thus, although modified purines are useful in certain respects, they are not as generally useful as pyrimidines and 7-deazapurines. Moreover, pyrimidines and 7-deazapurines useful in that invention must not be naturally substituted at the 5- or 7- positions, respectively. As a result, certain bases, such as thymine, 5-methylcytosine, and 5-hydroxymethylcytosine, are not useful. Preferred bases are cytosine, uracil, deazaadenine and deazaguanine.

A may be any moiety which has at least three carbon atoms and is capable of forming a detectable complex with a polypeptide when the modified nucleotide is incorporated into a double-stranded duplex containing either deoxyribonucleic or ribonucleic acid.

A therefore may be any ligand which possesses these properties, including haptens which are only immunogenic when attached to a suitable carrier, but are capable of interacting with appropriate antibodies to produce complexes. Examples of moieties which are useful according to EP-A-0 063 879 include: 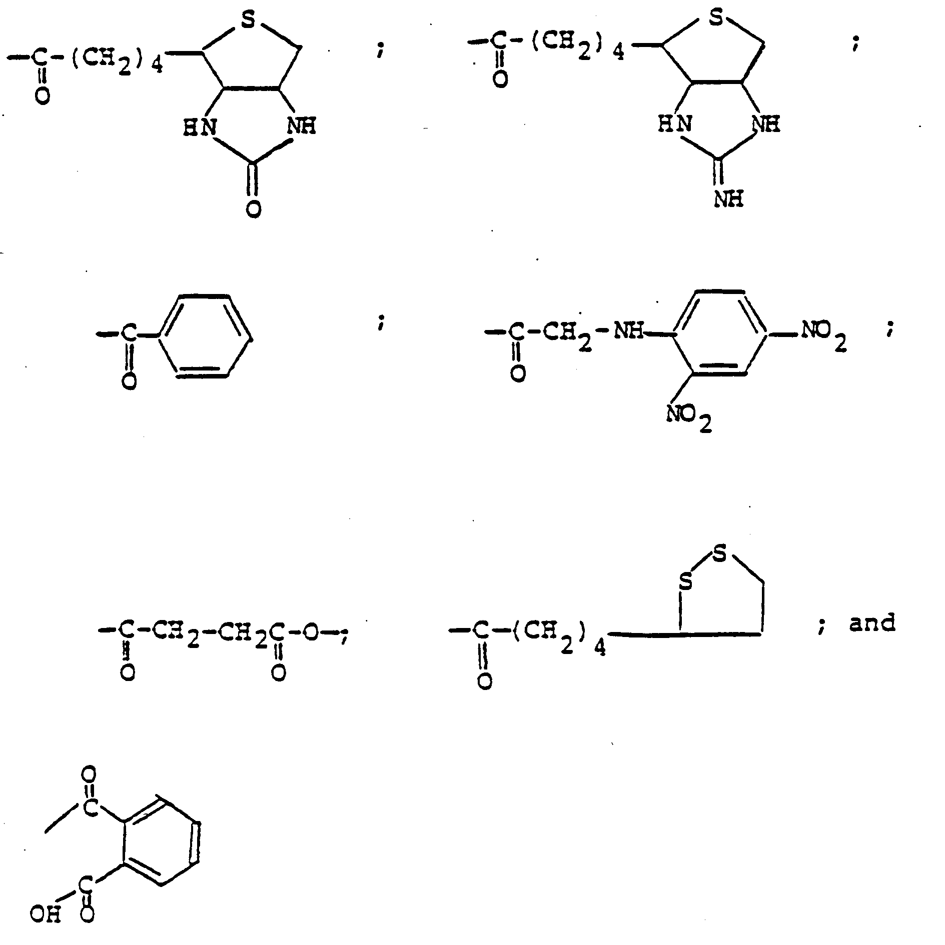
Of these, the preferred A moieties are biotin and iminobiotin.

Moreover, since aromatic moieties tend to intercalate into a base-paired helical structure, it is preferred in EP-A-0 063 879 that the moiety A be nonaromatic. Also, because smaller moieties may not permit sufficient molecular interaction with polypeptides, it is preferred that A be at least C₅ in structure so that sufficient interaction can occur to permit formation of stable complexes. Biotin and iminobiotin are said to satisfy both of these criteria.

According to EP-A-0 063 879 the linkage or group joining moiety A to base B may include any of the well known bonds, including carbon-carbon single bonds, carbon-carbon double bonds; carbon-nitrogen single bonds, or carbon-oxygen single bonds. However, it is generally preferred that the chemical linkage include an olefinic bond at the α-position relative to B. The presence of such an α-olefinic bond serves to hold the moiety A away from the base when the base is paired with another in the well known double-helix configuration. This permits interaction with polypeptide to occur more readily, thereby facilitating complex formation. Moreover, single bonds with greater rotational freedom may not always hold the moiety sufficiently apart from the helix to permit recognition by and complex formation with polypeptide.

According to EP-A-0 063 879 it is even more preferred that the chemical linkage group be derived from a primary amine, and have the structure -CH₂-NH-, since such linkages are easily formed utilizing any of the well known amine modification reactions. Examples of preferred linkages derived from allylamine and allyl-(3-amino-2-hydroxy-1-propyl) ether groups have the formulae -CH=CH-CH₂-NH- and 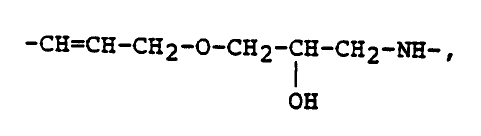
respectively.

Although these linkages are preferred, others can be used, including particularly olefin linkage arms with other modifiable functionalities such as thiol, carboxylic acid, and epoxide functionalities.

The linkage groups are attached at specific positions, namely, the 5-position of a pyrimidine, the 8-position of a purine, or the 7-position of a deazapurine. Substitution at the 8-position of a purine does not produce a modified nucleotide which is useful in all the methods discussed in EP-A-0 063 879. It may be that the 7-position of a purine, which is occupied by a nitrogen atom, could be the point of linkage attachment. However, the chemical substitution methods employed up to that time were not suitable for this purpose.

The letters x, y, and z represent groups attached to the 5', 3' and 2' positions of the sugar moiety. They may be any of

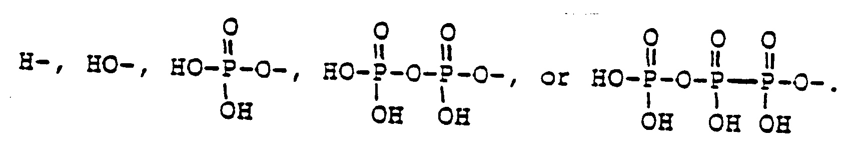

Although conceivable, it is unlikely that all of x, y, and z will simultaneously be the same. More likely, at least one of x, y, and z will be a phosphate-containing group, either mono-, di-,-or tri-phosphate, and at least one will be HO- or H-. As will be readily appreciated, the most likely identity of z will be HO- or H- indicating ribonucleotide or deoxyribonucleotide, respectively. Examples of such nucleotides include 5'-ribonucleoside monophosphates, 5'-ribonucleoside diphosphates, 5'-deoxyribonucleoside triphosphates, 5'p-ribonucleoside-3'p, and 5'p-deoxyribonucleoside-3'p. More specific examples include modified nucleotides of this type in which A is biotin or iminobiotin, the chemical linkage is -CH=CH-CH₂-NH- or 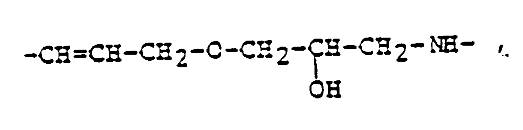
and B is uracil or cytosine.

The general synthetic approach adopted for introducing the linker arm and probe moiety onto the base is discussed thereinabove. (See especially, J.L. Ruth and D.E. Bergstrom, and M.K. Ogawa, J. Amer. Chem. Soc. 100, 8106, 1978; and C.F. Bigge, P. Kalaritis, J.R. Deck and M.P. Mertes, J. Amer. Chem. Soc. 102, 2033, 1980.). However, the olefin substituents employed herein have not been used previously. To facilitate attachment of probe moiety A, it is stated in EP-A-0 063 879 as being particularly desirable to employ olefins with primary amine functional groups, such as allylamine [AA] or allyl-(3-amino-2-hydroxy-1-propyl) ether [NAGE], which permit probe attachment by standard amine modification reactions, such as, 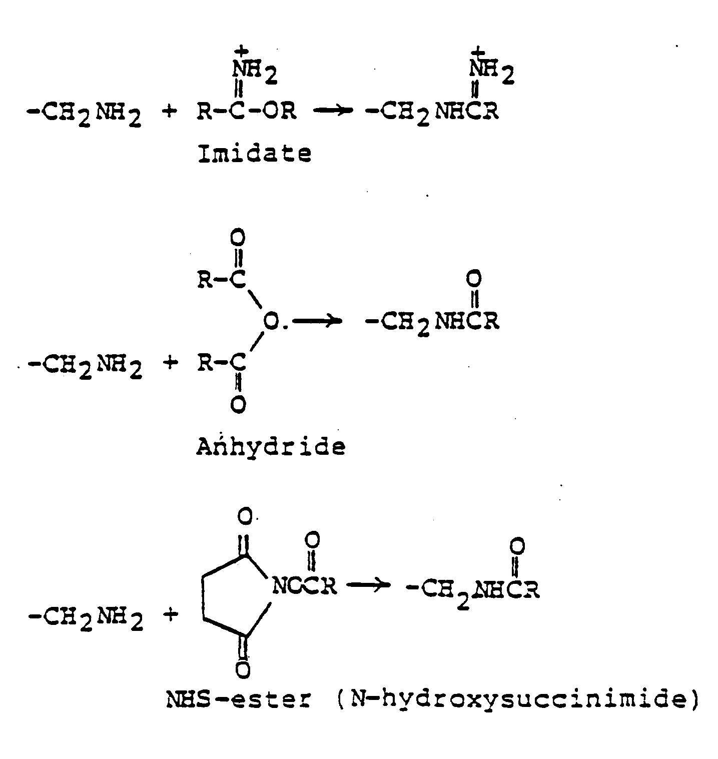 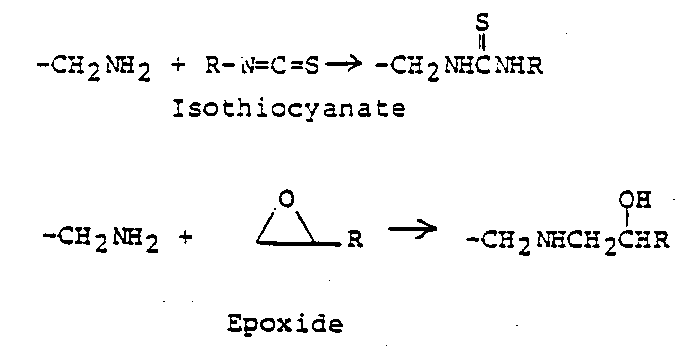
Because of ease of preparation, it is preferable according to EP-A-0 063 879 to use NHS-esters for probe addition. However, olefin linker arms with other modifiable functional groups, such as thiols, carboxylic acids, epoxides, and the like, can also be employed. Furthermore, both linker arm and probe can be added in a single-step if deemed desirable.

Having the biotin probe directly attached to the nucleotide derivatives that are capable of functioning as enzyme substrates offers considerable versatility, both in the experimental protocols that can be performed and in the detection methods (microscopic and non-microscopic) that can be utilized for analysis. For example, biotin nucleotides can be introduced into polynucleotides which are in the process of being synthesized by cells or crude cell extracts, thus making it possible to detect and/or isolate nascent (growing) polynucleotide chains. Such a procedure is impossible to do by any direct chemical modification method. Furthermore, enzymes can be used as reagents for introducing probes such as biotin into highly selective or site-specific locations in polynucleotides; the chemical synthesis of similar probe-modified products would be extremely difficult to achieve at best.

Further, there are disclosed in EP-A-0 097 373 modified non-radioactive chemically-labeled nucleotides wherein the nucleotides modified such as at the 5-position of pyrimidine or the 7-position of purine, preparatory for the preparation of nucleotide probes therefrom are suitable for attachment to or incorporation into DNA or other nucleic acid materials. In the preparation of such modified nucleotides, the nucleotides, i.e. nucleic acids, preferably are modified in a non-disruptive manner such that the resulting modified nucleotides are capable of incorporation into nucleic acids and once incorporated in nucleic acids, the modified nucleotides do not significantly interfere with the formation or stabilization of the double helix formed of the resulting nucleic acids containing the modified nucleotides. The non-disruptive modification of nucleotides and nucleic acids incorporating such modified nucleotides is in contrast with those modifications of nucleotides which are characterized as a disruptive modification in the sense that the resulting disruptively modified nucleotides and nucleic acids containing the same block proper double helix formation. In the practices of EP-A-0 097 373, the nucleotides are desirably modified at the 5-position of the pyrimidine or the 7-position of the purine. The nucleotides so modified are non-disruptively modified and nucleic acids containing such nucleotides are capable of forming a double helix arrangement.

Broadly, in another aspect of the practices of EP-A-0 097 373 , various methods are useful for the tagging or labeling of DNA in a non-disruptive manner. For example, biotin is added on the end of a DNA or RNA molecule. The addition of biotin is accomplished by addition of a ribonucleotide. The 3',4' vicinal hydroxyl groups are oxidized by periodate oxidation and then reduced by a borohydride in the presence of biotin hydrazide. Alternatively, carbodiimide can also be used to couple biotin to the aldehyde group.

Another technique for tagging nucleic acid material such as DNA or RNA involves the addition of a large marker to the end of a DNA or RNA molecule. One example of this technique is the addition of a molecule, e.g. lysylglycine, where the amino groups are tagged with biotin. Another example would be to follow the procedure set forth hereinabove but employing carbodiimide as the cross-linking agent. Still another example of this technique would be to produce a biotinylated dA:dU double helical polymer and to ligate this polymer to the probe prepared in accordance with EP-A-0 097 373.

Another technique for tagging DNA in a non-disruptive manner involves the isolation of dPyrTP having a putricine or spermidine on the 5-position from PS16 or phage-infected cells. If desired, dPyrTP is made from phage DNA and phosphorylated to dPyrTP followed by modification of the polyamine side chain by means of standard nucleophilic reagent NHS-biotin.

Another technique for tagging DNA in a non-disruptive manner involves the addition of glucose to 5-hydroxymethylcytosine (5 HMC) in DNA using T4 phage glycosylating enzymes followed by screening by means of a lectin-based assay.

Still another method for tagging DNA in a non-disruptive manner involves 5-HMC-triphosphate made from the hydrolysis of T4-DNA followed by phosphorylation of the 5HMCMP to 5 MMCTP. 5 HMCTP is then incorporated into DNA using polymerase I. Thus, any DNA can be modified to have non-disruptively incorporated therein 5 HMC.

A method for tagging DNA in a mildly disruptive manner according to EP-A-0 097 373 involves reacting nucleic acids in the double helical form with alkylating reagents as for example benz(o)pyrene diol epoxide or aflatoxin. Under appropriate conditions of the N² group of guanine, the N⁴ group of adenosine or the N⁴ group of cytosine are alkylated. These modified nucleotides can be used as linking arms for the addition of a reporter molecule such as biotin.

These specially modified nucleotides suitable as non-radioactive chemical labels for DNA probes or DNA material as described in EP-A-0 097 373 can be broadly characterized and described as phosphate P moiety, a sugar or monosaccharide S moiety, a base B moiety, a purine or a pyrimidine and a signalling chemical moiety Sig covalently attached thereto, either to the P, S or B moiety.

Of special interest are those nucleotides having a general formula

P - S - B - Sig

wherein P is the phosphate moiety including mono-, di-, tri-, or tetraphosphate, S the sugar or monosaccharide moiety, B the base moiety, either a purine or a pyrimidine. The phosphate moiety P is attached at the 3' and/or the 5' position of the S moiety when the nucleotide is a deoxyribonucleotide and at the 2', 3' and/or 5' position when the nucleotide is a ribonucleotide. The base B moiety is attached from the N1 position of the N9 position to the 1' position of the S moiety when the base moiety is a pyrimidine or a purine, respectively. The Sig moiety is covalently attached to the B moiety of the nucleotide and when so attached is capable of signalling itself or makes itself self-detecting or its presence known and desirably or preferably permits the incorporation of the resulting nucleotide P - S - B - Sig into or to form a double-stranded helical DNA or RNA or DNA-RNA hybrid and/or to be detectable thereon.

Another special nucleotide in accordance with EP-A-0 097 373 is characterized by the general formula: 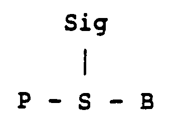
Such nucleotides would be characterized as ribonucleotides. The phosphate moiety is attached at the 2', 3' and/or 5' position of the sugar S moiety and the base B being attached from the N1 position or the N9 position to the 1' position of the sugar S moiety when said base is a pyrimidine or a purine, respectively. The Sig chemical moiety is covalently attached to the sugar S moiety is capable of signalling itself or making itself self-detecting or its presence known and preferably permits the incorporation of the ribonucleotide into its corresponding double-stranded RNA or a DNA-RNA hybrid.

Such nucleotides 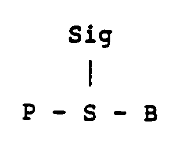
desirably have the Sig chemical moiety attached to the C2' position of the S moiety or the C3' position of the S moiety.

Still further, nucleotides in accordance with the practices of EP-A-0 097 373 include the nucleotides having the formula 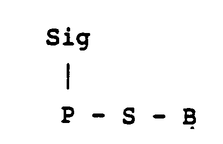
wherein P is the phosphate moiety, S the sugar moiety and B the base moiety. In these special nucleotides, the P moiety is attached to the 3' and/or the 5' position of the S moiety when the nucleotide is deoxyribonucleotide and at the 2', 3' and/or 5' position when the nucleotide is a ribonucleotide. The base B is either a purine or a pyrimidine and the B moiety is attached from the N1 or the N9 position to the 1' position of the sugar moiety when said B moiety is a pyrimidine or a purine, respectively. The Sig chemical moiety is covalently attached to the phosphoric acid P moiety via the chemical linkage 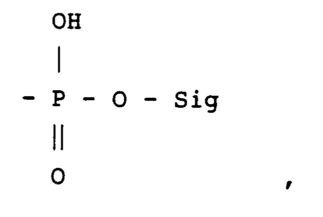
said Sig, when attached to said P moiety being capable of signalling itself or making itself self-detecting or its presence known and desirably the nucleotide is capable of being incorporated into a double-stranded polynucleotide, such as DNA, RNA or DNA-RNA hybrid and when so incorporated therein is still self-detecting.

It is pointed out that the special nucleotides in accordance with the practices of EP-A-0 097 373 described or defined hereinabove by the general formula P - S - B - Sig, also include nucleotides wherein the Sig chemical moiety is covalently attached to the B moiety at the N⁶ or 6-amino group position when the B moiety is adenine or the N² or 2-amino group position when the B moiety is guanine or the N⁴ or 4-amino group position when the B moiety is cytosine. The resulting nucleotides containing the Sig moiety attached thereto are capable of signalling themselves or making themselves self-detecting or their presence known and being detectable is a double-stranded or DNA, RNA or DNA-RNA hybrid.

By way of summary, as indicated hereinabove with respect to the make up of the various special nucleotides in accordance with EP-A-0 097 373, the special nucleotides can be described as comprising a phosphate moiety P, a sugar moiety S and a base moiety B, a purine or pyrimidine, which combination of P-S-B is well known with respect to and defines nucleotides, both deoxyribonucleotides and ribonucleotides. The nucleotides are then modified by having covalently attached thereto, to the P moiety and/or the S moiety and/or the B moiety, a chemical moiety Sig. The chemical moiety Sig so attached to the nucleotide P-S-B is capable of rendering or making the resulting nucleotide, now comprising P-S-B with the Sig moiety being attached to one or more of the other moieties, self-detecting or signalling itself or capable of making its presence known per se, when incorporated into a polynucleotide, especially a double-stranded polynucleotide, such as a double-stranded DNA, a double-stranded RNA or a double-stranded DNA-RNA hybrid. The Sig moiety desirably should not interfere with the capability of the nucleotide to form a double-stranded polynucleotide containing the special Sig-containing nucleotide in accordance with this invention and, when so incorporated therein, the Sig-containing nucleotide is capable of detection, localization or observation.

The Sig moiety employed in the make up of the special nucleotides of EP-A-0 097 373 could comprise an enzyme or enzymatic material, such as alkaline phosphatase, glucose oxidase, horseradish peroxidase or ribonuclease. The Sig moiety could also contain a fluorescing component, such as fluorescein or rhodamine or dansyl. If desired, the Sig moiety could include a magnetic component associated or attached thereto, such as a magnetic oxide or magnetic iron oxide, which would make the nucleotide or polynucleotide containing such a magnetic-containing Sig moiety detectable by magnetic means. The Sig moiety might also include an electron dense component, such as ferritin, so as to be available by observation. The Sig moiety could also include a radioactive isotope component, such as radioactive cobalt, making the resulting nucleotide observable by radiation detecting means. The Sig moiety could also include a hapten component or per se be capable of complexing with an antibody specific thereto. Most usefully, the Sig moiety is a polysaccharide or oligosaccharide or monosaccharide, which is capable of complexing with or being attached to a sugar or polysaccharide binding protein, such as a lectin, e.g. Concanavilin A. The Sig component or moiety of the special nucleotides could also include a chemiluminescent component.

As indicated in accordance with the practices of EP-A-0 097 373, the Sig component could comprise any chemical moiety which is attachable either directly or through a chemical linkage or linker arm to the nucleotide, such as to the base B component therein, or the sugar S component therein, or the phosphoric acid P component thereof.

The Sig component of the nucleotides in accordance with EP-A-0 097 373 and the nucleotides and polynucleotides incorporating the nucleotides containing the Sig component are equivalent to and useful for the same purposes as the nucleotides described in EP-A-0 063 879. More specifically, the chemical moiety A described in EP-A-0 063 879 is functionally the equivalent of the Sig component or chemical moiety of the special nucleotides of EP-A-0 097 373. Accordingly, the Sig component or chemical moiety of nucleotides of EP-A-0 097 373 can be directly covalently attached to the P, S or B moieties or attached thereto via a chemical linkage or linkage arm as described in EP-A-0 097 373, as indicated by the dotted line connecting B and A of the nucleotides of EP-A-0 063 879 . The various linker arms or linkages identified in EP-A-0 063 879 applicable to and useful in the preparation of the special nucleotides of EP-A-0 097 373.

A particularly important and useful aspect of the special nucleotides of EP-A-0 097 373 is the use of such nucleotides in the preparation of DNA or RNA probes. Such probes would contain a nucleotide sequence substantially matching the DNA or RNA sequence of genetic material to be located and/or identified. The probe would contain one or more of the special nucleotides of this invention. A probe having a desired nucleotide sequence, such as a single-stranded polynucleotide, either DNA or RNA probe, would then be brought into contact with DNA or RNA genetic material to be identified. Upon the localization of the probe and the formation of a double-stranded polynucleotide containing the probe and the matching DNA or RNA material to be identified, the resulting formed double-stranded DNA or RNA-containing material would then be observable and identified. A probe in accordance with EP-A-0 097 373 may contain substantially any number of nucleotide units, from about 5 nucleotides up to about 500 or more, as may be required. It would appear that 12 matching, preferably consecutive, nucleotide units would be sufficient to effect an identification of most of the DNA or RNA material to be investigated or identified, if the 12 nucleotide sequence of the probe matches a corresponding cooperative sequence in the DNA or RNA material being investigated or to be identified. As indicated, such probes may contain one or more of the special Sig-containing nucleotides preferably at least about one special nucleotide per 5-10 of the nucleotides in the probe.

The present invention relates to a method for detecting a polynucleotide sequence which comprises:
- fixing said polynucleotide sequence to a solid support which comprises or is contained within a transparent or translucent system, such that the polynucleotide is in a single-strand form and is capable of hybridizing to complementary nucleic acid sequences;
- forming an entity comprising said polynucleotide sequence hybridized to a polynucleotide or oligonucleotide probe, said probe having attached thereto a chemical label comprising a signalling moiety capable of generating a signal; and
- generating and detecting a signal, characterized in that the transparent or translucent system is a non-porous system and the generated signal is a soluble signal.

In accordance with the practice of this invention, non-radioactive chemically labeled polynucleotides are prepared such as non-radioactive chemically labeled single-stranded DNA probes. Such probes are then brought into contact with genetic material to be identified or otherwise detected. Before these probes are brought into contact with the genetic material to be identified or investigated, the genetic material is denatured to produce or form single-stranded genetic material therefrom, such as single-stranded DNA derived from the genetic material, and is fixed to a suitable inert support or surface such as a plastic material, e.g. polystyrene, preferably a transparent or translucent surface such as glass. Thereupon, the special non-radioactive chemically-labeled probes, such as the special non-radioactive chemically-labeled single-stranded DNA probes of this invention are brought into contact with the thus-fixed single-stranded genetic material under hybridizing conditions. The probe is selected to provide sufficient number, e.g. at least about 25 bases, in its make up, complementary to the bases making up the genetic material to be detected or identified. The hybridization of the probe to the singlestranded genetic material to be identified with resulting double-stranded or duplex formation would be then detected by means of the non-radioactive chemical Label attached to the probe portion of the resulting formed double-stranded hybrid or duplex hybrid. Various techniques, depending upon the non-radioactive chemical label employed in the make up of the probe, may be employed to detect the formation of the double strand or duplex hybrid. It is preferred, however, in the practices of this invention, to employ spectrophotometric techniques and/or enzyme linked immunosorbent assay (ELISA) techniques for the determination of the formed hybrid. Spectrophotometric and ELISA techniques permit not only the detection of the resulting formed double-stranded hybrid, but also permit the quantitative determination thereof, such as by the enzymatic generation of a product that can be measured colorimetrically or fluorometrically. In colorimetric determination, an enzyme linked antibody, such as alkaline phosphatase linked antibody, would be attached to the non-radioactive chemically-labeled probe and employed for the enzymatic generation of the product that can be measured colorimetrically or spectrophotometrically. Another suitable spectrophotometric or colorimetric technique involves immunofluorescence, wherein fluorescein-labeled antibody is employed in the make up prior to or is attached to the non-radioactive chemical label after hybrid formation and the fluorescein-labeled antibody attached or fixed to the probe measured fluorometrically.

Spectrophotometric or colorimetric techniques, in addition to providing the above-identified quantitative result, also usefully provide a fairly prompt visual manifestation or elicitation of the non-radioactive chemical label in the resulting formed double-stranded hybrid. Other ELISA-like or related techniques are also useful for the detection of the non-radioactive chemical label in the formed duplex, such other techniques would also involve the use of enzymes, e.g. immunoperoxidase, or the use of electron dense markers, e.g. immunoferritin, or other chemical and/or physical markers, attachable or attached to the probes. Broadly, the practices of this invention provide techniques comparable to enzyme linked immunosorbent assay techniques, not only for the qualitative, but also the quantitative determination of hybrid formation.

As indicated, it is preferred to use ELISA techniques to effect or bring about the expression or indication of, qualitatively or quantitatively, of the non-radioactive chemically-labeled probe in the resulting formed double-stranded or hybridized material, such as the duplex or hybrid formed by the non-radioactive chemically-labeled DNA probe and its substantially complementary genetic DNA material derived from the denatured genetic DNA material to be identified. In the preferred ELISA technique in accordance with this invention, single-stranded genetic material, e.g. DNA, after denaturing of the DNA material to be identified to form the single-stranded DNA material, is fixed to a substrate. In the practices of this invention, it is preferred that the substrate be a transparent substrate, such as a glass substrate or surface. By employing a transparent substrate, such as a glass substrate, to which the single-stranded DNA material is fixed and hybridized, the ELISA technique provides for qualitative and quantitative determination of the DNA material to be identified. By employing a transparent substrate to which the DNA material is fixed, full realization of the benefits and versatility of the ELISA techniques applied to the practices of this invention are obtainable.

Further embodiments of the present invention are explained in detail in the following:
A transparent or translucent solid, non-porous substrate having fixed thereto a double-stranded polynucleotide, one of the strands of said double-stranded polynucleotide being a non-radioactive chemically labeled polynucleotide or comprising a non-radioactive chemically-labelled nucleotide as a nucleotide component of said one strand, wherein said chemically labelled polynucleotide comprises or has attached thereto a chemical label comprising a signalling moiety which generates a soluble signal which is detected spectrophotometrically.

The substrate may be made of glass or other siliceous material.

Said chemically-labeled nucleotide may be a compound of the formula: 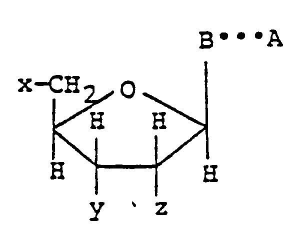
wherein B represents a purine, 7-deazapurine or pyrimidine moiety covalently bonded to the C^{1'}-position of the sugar moiety, provided that when B is purine or 7-deazapurine, it is attached at the N⁹-position of the purine or deazapurine, and when B is pyrimidine, it is attached at the N¹-position;
wherein A represents a moiety consisting of at least three carbon atoms which is capable of forming a detectable complex with a polypeptide when the compound is incorporated into a double-stranded ribonucleotide acid, deoxyribonucleic acid duplex, or DNA-RNA hybrid;
wherein the dotted line represents a linkage or group joining B and A, provided that if B is purine, the linkage is attached to the 8-position of the purine, if B is 7-deazapurine, the linkage is attached to the 7-position of the deazapurine, and if B is pyrimidine, the linkage is attached to the 5-position of the pyrimidine; and
wherein each of x, y and z represents 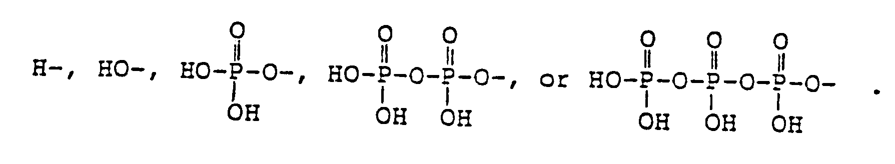
or of the formula, 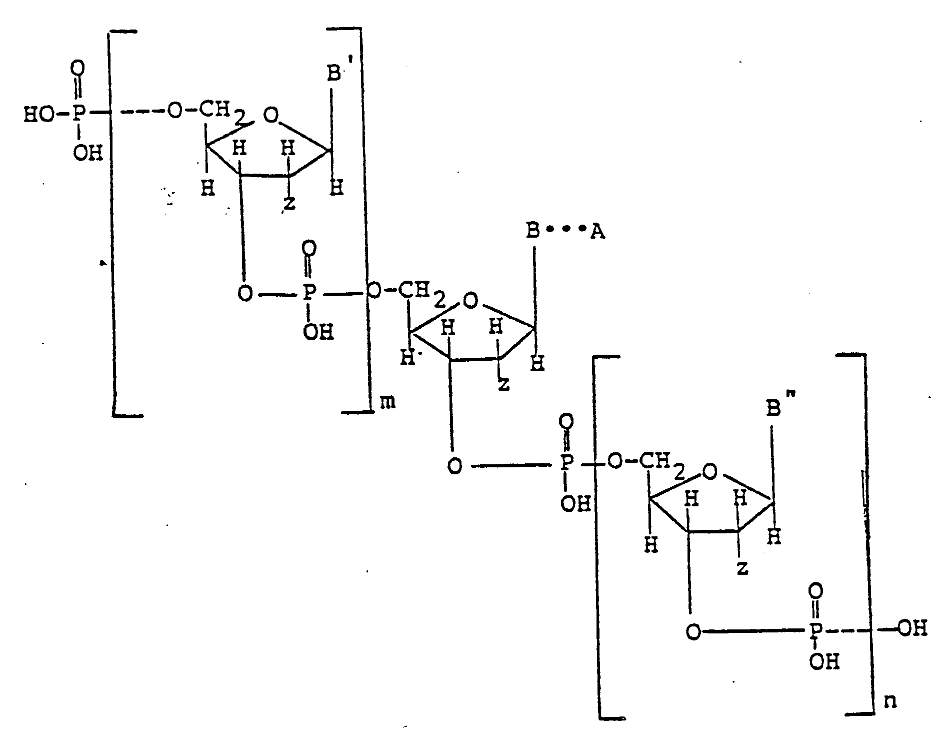
wherein each of B, B' and B'' represents a purine, deazapurine, or pyrimidine moiety covalently bonded to the C^{1'}-position of the sugar moiety, provided that whenever B, B' or B'' is purine or deazapurine, it is attached at the N⁹-position of the purine or deazapurine, and whenever B, B' or B'' is pyrimidine, it is attached at the N¹-position;
wherein A represents a moiety consisting of at least three carbon atoms which is capable of forming a detectable complex with a polypeptide when the compound is incorporated into a double-stranded duplex formed with a complementary ribonucleic or deoxyribonucleic acid molecule;
wherein the dotted line represents a chemical linkage or group joining B and A, provided that if B is purine, the linkage is attached to the 8-position of the purine, if B is 7-deazapurine, the linkage is attached to the 7-position of the deazapurine, and if B is pyrimidine, the linkage is attached to the 5-position of the pyrimidine;
wherein Z represents H- or HO-; and
wherein m and n represent integers from 0 up to about 100,000.

The chemically-labeled nucleotide has the general formula,

P-S-B-Sig

wherein P is the phosphate moiety, S the sugar or monosaccharide moiety, B being the base moiety, the phosphoric acid moiety being attached at the 3' and/or the 5' position of the sugar moiety when said nucleotide is a deoxyribonucleotide and at the 2', 3' and/or 5' position when said nucleotide is a ribonucleotide, said base being a purine or a pyrimidine, said base being attached from the N1 position or the N9 position to the 1' position of the sugar moiety when said base is a pyrimidine or a purine, respectively, and wherein said Sig is a chemical moiety covalently attached to the base B of said nucleotide, said Sig when attached to said base B being capable of signalling itself or making itself self-detecting or its presence known. or the general formula, 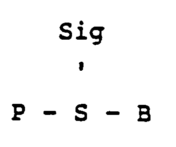
wherein P is the phosphate moiety, S the sugar moiety and B the base moiety, the phosphate moiety being attached at the 2', 3' and/or 5' position of the sugar moiety, said base B being attached from the N1 position or the N9 position to the 1' position of the sugar moiety when said base is a pyrimidine or a purine, respectively, and wherein said Sig is a chemical moiety covalently attached to the sugar S, said Sig, when attached to said Sugar S, being capable of signalling itself or making itself self-detecting or its presence known; or the general formula, 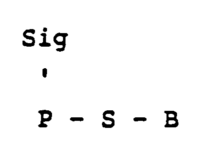
wherein P is the phosphate moiety, S the sugar moiety and B the base moiety, the phosphate moiety being attached to the 3' and/or the 5' position of the sugar moiety when said nucleotide is a deoxyribonucleotide and at the 2', 3' and/or 5' position when said nucleotide is a ribonucleotide, said base B being a purine or pyrimidine, said base B being attached from the N1 position or the N9 position to the 1' position of the sugar moiety when said base B is a pyrimidine or a purine, respectively, and wherein Sig is a chemical moiety covalently attached to the phosphate moiety via the chemical linkage, 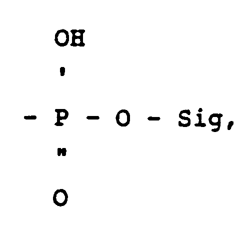
said Sig, when attached to said phosphate moiety P being capable of signalling itself or making itself self-detecting or its presence known, or the general formula,

P - S - B - Sig

wherein P is the phosphate moiety, S the sugar and monosaccharide moiety, B being the base moiety, the phosphate moiety being attached to the 3' and/or the 5' position of the sugar moiety when said nucleotide is deoxyribonucleotide and at the 2', 3' and/or 5' position when said nucleotide is a ribonucleotide, said base being a purine or a pyrimidine, said base being attached from the N1 position or the N9 position to the C1' position of the sugar moiety when said base is a pyrimidine or a purine, respectively, and wherein said Sig is a chemical moiety covalently attached to the base B of said nucleotide, said Sig being attached to the N⁶ or 6-amino group when said base B is adenine or the N² or 2-amino group when said base B is guanine or the N⁴ or 4-amino group when said base B is cytosine, said Sig when attached to said base B being capable of signalling itself or making itself self-detecting or its presence known, or the general formula,

P-S-B,

wherein P is the phosphate moiety, S the sugar or monosaccharide moiety and B the base moiety, said nucleotide having covalently attached to the P or S or B moiety a chemical moiety Sig, said Sig chemical moiety when attached to the P moiety, is attached thereto via the chemical linkage, 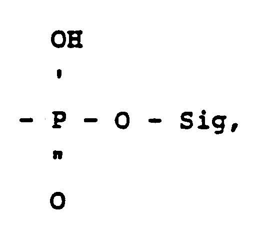
and when Sig is attached to the S moiety, the S moiety is a ribose group, said chemical moiety Sig when attached to said P, S or B being capable of signalling itself or making itself self-detecting or its presence known.

The method of the present invention may be used for detecting the presence of a pathogen in a clinical sample suspected of containing said pathogen, said method comprising depositing said sample on an inert transparent or translucent support, treating said sample to affix genetic material of any of said pathogens present in said sample to said support in substantially single-stranded form, contacting said fixed single-stranded genetic material with a non-radioactive chemically-labeled probe having a nucleotide sequence of at least about 25 bases at least substantially complementary to a nucleotide sequence of a structural gene characteristic of said pathogen, said chemically labeled probe having therein one of the chemically labeled nucleotides as described above, said contacting being under hybridizing conditions at a predetermined stringency and detecting duplex formation on said support by means of said chemically labeled probe.

The above-mentioned substrate is further characterized or may be modified in the following ways;
A substrate wherein said substrate contains said double-stranded polynucleotide attached thereto, said double-stranded polynucleotide comprising said single-stranded non-radioactive chemically labeled polynucleotide or nucleotide, the other strand being unlabeled single-stranded polynucleotide, said substrate being prepared by fixing said other single-stranded unlabeled polynucleotide to said substrate and then hybridizing to said unlabled polynucleotide said one strand containing said chemically labeled polynucleotide or nucleotide.

A substrate wherein said single-stranded non-radioactive chemically labeled polynucleotide contains at least 25 bases which are substantially complementary to the bases making up the other unlabeled single-stranded polynucleotide of said doublestranded polynucleotide.

A substrate wherein said chemically labeled polynucleotide is a polynucleotide coupled to or attached to a polypeptide.

A substrate wherein said chemically labeled polynucleotide is a polynucleotide coupled to or attached to a polypeptide, said polypeptide being terminally attached to or ligated to said polynucleotide.

A substrate wherein said chemically labeled polynucleotide comprises or has attached thereto an amino acid or polypeptide comprising a Sig chemical moiety covalently attached thereto, said Sig chemical moiety being capable of signalling itself or making itself self-detecting or its presence known, whereby said Sig chemical moiety comprises a saccharide component, or includes a co-enzyme, wherein said co-enzyme is selected from the group consisting of thiamine pyrophosphate, flavine mononucleotide, flavine adenine dinucleotide, nicotinamide adenine dinucleotide, nicotinamide adenine dinucleotide phosphate, co-enzyme A, pyridoxyl phosphate, biotin, tetrahydrofolic acid, coenzyme B₁₂, lipoic acid and ascorbic acid.

A substrate wherein said chemically labeled polynucleotide comprises or has attached thereto a monosaccharide or a polysaccharide comprising a Sig chemical moiety attached thereto, said Sig chemical moiety being capable of signalling itself or making itself self-protecting or its presence known, wherein said Sig chemical moiety comprises a chelating agent, or wherein said Sig chemical moiety includes a co-enzyme, wherein said co-enzyme is selected from the group consisting of thiamine pyrophosphate, flavine mononucleotide, flavine adenine dinucleotide, nicotinamide adenine dinucleotide, nicotinimide adenine dinucleotide phosphate, co-enzyme A, pyridoxyl phosphate, biotin, tetrahydrofolic acid, co-enzyme B₁₂, lipoic acid and ascorbic acid.

The double-stranded polynucleotide may be a double-stranded polyribonucleotide, or a double-stranded polydeoxyribonucleotide, or it comprises as one of the strands polydeoxyribonucleotide and the other strand a polyribonucleotide.

The substrate may be transparent or translucent and adapted for the transmission of light therethrough for color observation or colorimetric determination of said double-stranded polynucleotide on said transparent substrate, whereby said transparent substrate is glass.

Said substrate may be a planar substrate provided with a well therein wherein said double-stranded polynucleotide is fixed, whereby said substrate carrying said double-stranded polynucleotide fixed to a well therein is adapted for the transmission of light therethrough for the photometric or colorimetric determination of the double-stranded polynucleotide fixed within said well. Said substrate is a plastic-coated substrate, a glass-coated substrate, a plastic substrate, a polystyrene substrate, a polyethylene substrate, a polypropylene substrate, a celluose substrate, a nitrocellulose substrate, a dextran substrate, an epoxy or expoxy-coated substrate, has amino groups attached or fixed to the surface thereof, or provides a surface capable of fixing or immobilizing negatively charges polyelectrolytes thereon.

Said substrate provides a surface capable per se or untreated of fixing or immobilizing single-stranded or double-stranded polynucleotides thereto or permits the hybridization of a non-radioactive chemically-labeled polynucleotide to an unlabled single-stranded polynucleotide or DNA fixed to said surface.

The method of the present invention may be used for determining the presence of a selected genetic material which comprises treating a sample containing said selected genetic material so as to denature the same to provide a single strand thereof derived from said genetic material, fixing the resulting single strand of genetic material to a surface, contacting under hybridizing conditions the thus-fixed single strand of genetic material with a non-radioactively chemically-labeled polynucleotide probe substantially complementary to the nucleotide components characterizing said genetic material to be determined and detecting duplex formation between said single strand of genetic material and said non-radioactive chemically-labeled polynucleotide probe, wherein said surface is a glass surface and wherein said single-stranded genetic material is fixed to a well provided on said surface, said surface being adapted for the transmission of light therethrough for the photometric or colorimetric determination of duplex formation between said single-stranded genetic material and said polynucleotide probe, wherein said duplex formation between said single strand of genetic material and said non-radioactive, chemically-labeled polynucleotide probe is detected or determined by enzyme linked immunosorbent assay (ELISA).

The detection of duplex formation between said single strand of genetic material and said non-radioactive, chemically-labeled polynucleotide probe is determined by forming a complex with a chemical label provided on said polynucleotide probe, said complex comprising an enzyme, and eliciting the presence of said enzyme-containing complex attached to said chemical label by bringing said enzyme-containing complex attached to said polynucleotide probe into contact with a substrate which undergoes a chemical or color change when in contact with said enzyme.

The present invention further relates to a method wherein duplex or double strand formation or hybridization between said fixed single strand of material and said non-radioactive chemically-labeled polynucleotide probe is determined by providing said chemical label attached to said polynucleotiode probe, before or after duplex formation, with a chelating agent and eliciting the presence of said chelating agent by contacting said chelating agent with a substrate which undergoes a chemical reaction or color change when in contact with said chelating agent, whereby eliciting the presence of said chelating agent is carried out by contacting said chelating agent with a substrate which undergoes a chemical reaction or a color change when in contact with said chelating agent, whereby said color change is photometrically or colorimetrically determined and is indicative of the amount of chelating agent fixed to the resulting formed duplex.

The support for fixing genetic DNA material thereto may be a glass surface which has been prepared by treating it with aqueous nitric acid solution at a temperature of about the boiling point of the nitric acid solution, washing the resulting nitric acid-treated glass surface and, after drying the thus-treated glass surface, contacting the thus-treated glass surface with gamma-aminopropyltriethoxysilane, washing and drying the resulting treated glass surface and fixing DNA material thereto, whereby the transparent glass surface suitable for fixing genetic DNA material thereto may have attached or fixed thereto amino groups.

Further methods for treating the surface are the following:
A method of preparing a surface selected from the group consisting of a glass surface or a plastic surface for the fixing of genetic material thereto by contacting said surface with the amine and the hardener components of an epoxy glue to form a coating of epoxy glue thereon.

A method of treating a glass surface for the fixing of genetic DNA material thereto, which comprises contacting said glass surface with an organosilane, said organosilane providing an organic functional group at one end and a silyl alkoxy group at the other end under conditions so as to fix the silyl alkoxy group portion of the organosilane to said glass surface.

In the method of the invention there may be used a transparent glass container or cuvette provided with planar walls, the interior surface of the container having fixed thereto double-stranded polynucleotide or a planar form transparent glass plate provided with rows of wells or depressions formed on the surface thereof, the surface of said wells or depressions having fixed thereto double-stranded polynucleotide, one of the strands of said double-stranded nucleotide being a non-radioactively chemically-labeled polynucleotide or comprises a non-radioactively chemically-labeled nucleotide as a nucleotide component of said one strand.

The above-mentioned substrate may be a cuvette having a planar surface adapted for the transmission of light therethrough, or the substrate is adapted for the transmission of light perpendicularly or transversely through the system.

If the method of the invention is used for determining the presence of genetic material, the detection of duplex formation by the said single-strand of genetic material and said non-radioactive, chemically-labeled polynucleotide probe is determined by forming a complex with a chemical label providing on said polynucleotide probe, said complex comprising a component capable of signalling or eliciting its presence by spectrophotometric or colorimetric means.

If the method of the invention is used for the detection of a pathogen, it may be a streptococcus, a staphylococcus, a pneumococcus, a meningococcus, a salmonella typhimurium, or a clostridium botulinum.

A specific embodiment of the claimed method is a method for detecting the presence or identity of genetic DNA material which comprises depositing a sample of genetic material on an inert transparent support surface, treating said sample to affix said genetic DNA material to said support in substantially single-stranded form, contacting said fixed single-stranded genetic DNA material with a non-radioactive chemically-labeled probe having a nucleotide sequence of at least about 25 bases, at least substantially complementary to the nucleotide sequence contained in said genetic DNA material, said chemically-labeled probe having therein a chemically-labled nucleotide as described above, said contacting being under hybridising conditions at a predetermined stringency and detecting duplex formation of said genetic DNA material fixed to said support surface by means of said chemically-labeled probe.

The invention also relates to a device or system for detecting or measuring a color change or chemical reaction colorimetrically or photometrically which comprises a transparent substrate which has associated with or fixed thereto a single stranded chemically labeled DNA probe hybridized to a complementary single stranded DNA material, said chemical label having an enzyme complex attached thereto, means for adding a chemical substrate to contact said enzyme complex attached to said chemical label of said hybridized single stranded DNA probe associated with or fixed to said transparent substrate so as to generate by reaction between said enzyme complex and said chemical substrate a color change or a photometrically detectable chemical reaction, means for illuminating or passing light onto or through said transparent substrate containing said enzyme complex involved in said color change or chemical reaction comprising means for colorimetrically or photometrically detecting said color change or photometrically detectable chemical reaction brought about by contact between said enzyme complex and said chemical substrate, wherein said means for illuminating or passing light onto or through said transparent substrate containing said enzyme complex involved in said color change or said chemical reaction includes means for quantitatively measuring said color change or said photometrically detectable chemical reaction.

The device or system may be packaged in association with instructions for use in accordance with the claimed method.

The invention further relates to a kit for detecting a polynucleotide sequence, which comprises the above-mentioned device in packaged combination with a container of an oligonucleotide or polynucleotide probe, having covalently attached thereto a chemical label comprising a signalling moiety capable of generating a soluble signal.

It is known to fix enzymes and the like to siliceous materials and to detect antibodies and the like involving attachments of such materials as enzymes, antibodies and antigens to various substrates, such as siliceous substrates. See for example U.S. Patent Nos. 3,669,841, 3,715,278, 3,949,064, 4,001,583, 4,059,685, 4,120,945 and 4,280,992. In the practices of this invention, it is highly desirable that the fixing of the denatured single-stranded genetic DNA material derived from the DNA material to be identified be rapidly fixed to the substrate, such as a transparent substrate, e.g. a glass surface. Rapid fixing of single-stranded DNA material to a transparent glass substrate would permit rapid testing of numerous samples involving ELISA techniques. For example, glass plates provided with an array of depressions or wells therein would have samples of the various denatured genetic materials to be identified deposited therein and the single-stranded DNA material therein fixed to the surfaces of the wells. Thereupon, DNA probes provided with a non-radioactive chemical label are deposited in each of the wells for hybridization to any complementary single-stranded DNA material therein. After washing to remove any non-hybridised probe, the presence of any hybrid DNA material containing the single-stranded DNA material to be identified and the non-radioactive chemically-labeled probe is detected, as described herein, involving the addition of an enzyme linked antibody or other suitable entity for attachment to the chemical label of the probe. Subsequently a suitable substrate is added to elicit a color change or chemical reaction which could then be measured colorimetrically or photometrically.

To effect rapid fixing of DNA material, such as denatured single-stranded DNA, to glass, the glass is prepared or pretreated by heating or boiling the glass, such as a borosilicate glass, for a sufficient period of time, e.g. about 45 minutes, in the presence of dilute, e.g. 5 percent, aqueous nitric acid. This pretreatment with nitric acid serves to leach out boron residues from the glass surface. The treated glass is then washed or rinsed with water, preferably with distilled water, and dried, such as at a temperature of about 115^{o}C, for about 24 hours. A 10 percent solution of gamma-aminopropyltriethoxysilane prepared by dissolving the above-identified silane in distilled water followed by addition of 6N hydrochloric acid to a pH of about 3.45, is then applied to the glass surface, and the glass surface is then incubated in contact with the above-identified silane solution for about 2-3 hours at a temperature of about 45^{o}C. The glass surface is then washed with an equal volume of water and dried overnight at a temperature of about 100^{o}C. The resulting treated glass surface has available alkylamine provided on the surface thereof suitable for immobilizing or fixing any negatively charged polyelectrolytes applied thereto. In connection with the above, see Weetal, H.H. and Filbert, A.M., "Porous Glass for Affinity Chromatography Applications", Methods in Enzymology, Vol. XXXIV, Affinity Techniques Enzyme Purification: Part B, pp. 59-72, W.B. Jakoby and M. Wilchek, eds.

To illustrate the practices of this invention, various DNA materials were prepared. For example, bacteriophage T₄ DNA was isolated from E. coli CR63 cultures infected with phage T₄ AM82 [44⁻62⁻] and purified to be free of chromosomal DNA. Highly purified calf thymus DNA was obtained from a biological supply service. Bacteriophage lambda was obtained by heat induction of E. coli strain W3350 lysogenic for λC₁ 857 phage and was employed for the preparation of phage lambda DNA. Also, radioactive DNA was prepared by nick translation with [³H] dATP and phage lambda DNA was also nick translated with maltose-triose dUTP to introduce glucosyl or saccharide residues into the DNA.

Other reagents were obtained or prepared, for example, lectins which readily and eagerly form a complex with glucosyl groups. Specifically Concanavalin A [ConA] was obtained and solubilized in 2.0M NaCl at a concentration of 50 mg/ml. Also, fluorescein-labeled ConA was prepared by reacting ConA with fluorescein isothiocyanate at a FITC to protein molar ratio of 3 in 0.1M sodium borate solution at a pH of 9.2 and at a temperature of 37^{o}C for 60 minutes. Any unreacted FITC was removed by gel filtration on Sephadex G-50.

A glass surface treated as described hereinabove was employed in the detection of lectin-binding to glucosylated DNA. In this procedure, glucosylated DNA [T₄ DNA] or non-glucosylated DNA [calf thymus DNA ] was delivered in 100 µl portions to treated glass tubes in triplicate set. After 15-30 minutes at room temperature, the solution was removed and the tubes rinsed generously with PBS·Mg⁺⁺ buffer [100mM Na-K-Po₄, pH 6.5, 150mM NaCl and 10mM MgCl₂]. One set of tubes was checked for the presence of DNA by staining with ethidium bromide [100 µl of 1 mg/ml solution, 30 minutes in the dark, at room temperature]. The staining solution was removed and the tubes rinsed and checked under UV light. To another set of tubes there was delivered fluorescent-labeled ConA [100 µl of 0.1 mg/ml in PBS·Mg⁺⁺ buffer]. After 60 minutes at room temperature, the solution was removed and the tubes were rinsed and checked under UV light.

To the third set of tubes was delivered 100 µl of unlabeled ConA in PBS·Mg⁺⁺ buffer. After 60 minutes at room temperature, the tubes were rinsed free of ConA with 0.2M Imidazole buffer pH 6.5.

Acid phosphatase was then added [0.005 units in 100 µl at 0.2 percent phosphatase-free BSA] and the tubes were incubated at room temperature for 30 minutes. After rinsing with 0.15M NaCl (to remove any unbound enzyme) substrate [para-nitrophenylphosphate 0.1mM in 0.2M Imidazole pH 6.5] was added and incubation continued for 60 minutes at 37^{o}C. The enzyme reaction was terminated by adding 1.0 ml of 0.5 percent sodium bicarbonate and absorbance was determined at A₃₀₀.

The resulting observed test results indicated that both glucosylated and non-glucosylated DNA bound to the activated glass surface by the observed red fluorescence characteristic of ethidium bromide. Also, ConA bound only to glucosylated DNA as noted by the green fluorescence in tubes containing T₄ DNA, but not in tubes which had calf thymus DNA. Further, acid phosphatase, which is a glycoprotein, gave positive reaction only in tubes containing T₄ DNA and ConA, but was washed off from the tubes which contained only ConA or ConA and calf thymus DNA.

Activated glass tubes were also employed in the detection of lectin-binding to glucosylated DNA probes hybridized to DNA already immobilized on glass surface. In these test, phage lambda DNA was immobilized on glass surface. After rinsing with buffer, the tubes were coated with 100 µl of coating solution [50 percent formamide, 5X SSC, 100 µg salmon sperm DNA, 0.2 percent polyvinyl pyrrolidone, 0.1 percent Triton X-100, 0.2 percent BSA and 0.05 percent SDS] at 42^{o}C for 90-120 minutes. The coating solution was removed and the surface was covered with 100 µl of coating solution containing glucosylated nick-translated DNA probe. The probe had been denatured at 80^{o}C for 3 minutes and rapidly cooled in ice bath immediately before use. The tubes were then incubated at 42^{o}C for 24 hours. The solution was removed and tubes were rinsed with PBS·Mg⁺⁺ buffer. The lectin-enzyme detection system was delivered as described before. The results indicated that acid phosphatase was not washed off from the tubes which contained glucosylated DNA probe, whereas, tubes containing non-glucosylated probe did not show any enzyme activity.

In these tests, the glucosyl (monosaccharide) moiety of the glucosylated DNA probe serves the non-radioactive chemical label and reacts with or is strongly attracted to the lectin, ConA, making up the combination lectin-enzyme (acid phosphatase) probe detection system.

In the above test employing glucosylated DNA as a probe, wherein the glucosyl or monosaccharide moiety of the glucosylated DNA probe serves as the non-radioactive chemical label, comparable results are also achievable in the practice of this invention employing a biotin-labeled DNA probe. When biotin is employed as the non-radioactive chemical label of the DNA probe, and since avidin is strongly reactive with or strongly bonds to biotin, the presence of the biotin-labeled DNA probe would be elicited or detected by means of avidin or streptavidin-labeled enzyme. For example, a biotin-labeled DNA probe would readily be detected by an enzyme complex of the character avidin-biotin-alkaline phosphatase. More specifically, the presence of the biotin-labeled DNA probe would readily be elicited or detected by contacting the hybrid containing the biotin-labeled probe with the enzyme complex avidin-biotin-alkaline phosphatase, followed by bringing the resulting biotin-labeled DNA probe having attached thereto the avidin-biotin-alkaline phosphatase complex into contact with a suitable substrate which by color reaction or precipitate formation brought about by the alkaline phosphatase would be readily noticed or capable of being determined, both qualitatively and quantitatively, by photometric and/or colorimetric means in accordance with ELISA techniques. If desired, instead of an avidin-biotin-enzyme complex, there could be used an antibody to biotin for attachment to the biotin moiety of the biotin-labeled DNA probe, followed by a complex comprising anti-antibody-enzyme in the manner described hereinabove.

The advantages of the practices of this invention are also obtainable when the DNA probe is fixed to or hybridized to a plastic surface, such as a polystyrene surface. When a plastic surface is employed, it is sometimes desirable, in order to increase the effectiveness or uniformity of the fixing of DNA applied thereto, including the non-radioactive chemically-labeled DNA probe, to treat the plastic surface, such as a polystyrene surface, to enhance and otherwise improve the fixing or immobilization of genetic material thereon, such as single-stranded denatured DNA or a non-radioactive chemically-labeled probe or hybrid DNA containing the DNA probe. It has been found, for example, that the adherence or fixing of DNA to a polystyrene surface is improved by treating the surface, as indicated hereinabove, with an amino-substituted hydrophobic polymer or material, such as duodecadiamine. Another technique for improving the fixing or uniformity of the plastic surface for fixing DNA involves treatment of the surface with polylysine (PPL).

In tests involving the fixing of DNA to a plastic surface, biotinylated DNA was denatured and aliquoted into Dynatech, Immulon II™ removeable wells. Samples were allowed to dry onto the plastic surface at 37^{o}C. The amount of bound b-DNA was determined by sequential addition of goat anti-biotin antibody and rabbit anti-goat antibody complexed to alkaline phosphatase, followed by development with p-nitrophenyl phosphate in diethanolamine buffer, pH 9.6. Enzymatic activity was monitored at 405 nm utilizing the automatic Dynatech Micro ELISA Scanner. This procedure enables the investigator to quantitate the amount of bound DNA and circumspectively the degree of biotinylation.

To increase the sensitivity of detection, a fluorogenic substrate, such as 4-methylumbelliferyl-phosphate, or its analogues, with companion enzymes, may be used.

Additional tests were carried out wherein several aliquots of denatured adenovirus 2 DNA were bound to polystyrene plates as described above. After blocking with Denhardt's formamide blocking buffer, several biotinylated probes were hybridized to the immobilized DNA; these were B-adeno-2-DNA and lambda DNA. To one set of immobilized DNA, no probe was added. The extent of hybridization was determined by means of the antibody-enzyme reaction as described above. It was observed that only the homologous adeno-2 probe hybridized. This technique demonstrated that in vitro hybridization under these conditions is specific and can be monitored quantitatively.

Polystyrene from various batches or sources exhibits different binding capacities. Previous experiments had demonstrated that addition of duodecadiamine (DDA) to polystyrene resulted in an uniform binding coefficient of polystyrene plates of different batches.

In further tests, radioactively-labeled, non-biotinylated denatured DNA [2000 ng to 5 ng] denatured was applied to DDA-coated polystyrene plates. The test samples or plates were not allowed to dry. After incubation at 37^{o}C for 30 minutes, 1 hour, 2 hours, 3 hours, 4 hours, and 18 hours, samples were counted. Binding was maximal after 2 hours of incubation, however, 50 percent of the originally applied DNA bound regardless of the concentration, thereby indicating that there is an equilibrium between bound and unbound DNA.

In other tests, polystyrene microfilter wells were nitrated using a procedure of Filipsson and Hornby [Biochem J. 120 215 (1970)]. The polystyrene wells were immersed for 20 minutes in a mixture of concentrated nitric and sulfuric acid [41 percent, v/v] cooled to 0^{o}C. The wells were then washed thoroughly with water and subsequently heated to 70^{o}C in a 6 percent solution of sodium dithionate in 2M potassium hydroxide. After 4 hours, the wells were washed thoroughly with 0.5M hydrochloric acid and distilled water.

To produce 6-aminohexane linked polystyrene, 6-aminocaproic acid-N-hydroxysuccinimide ester·hydrobromide [5 mg thereof dissolved in 0.2M dimethylformamide prepared by reacting 6-aminocaproic acid·hydrobromide with N-hydroxysuccinimide and dicyclohexyl carbodiimide in dimethylformamide and recrystallized from isopropylalcohol] was added to 0.1M sodium borate [0.4 ml]. Amino-derivitized polystyrene microfilter wells filled with this solution were allowed to react at room temperature for 4 hours and then washed thoroughly with distilled water. The resulting treated wells absorbed ³H-labeled DNA from aqueous solution at pH less than 9.5.

As indicated hereinabove, siliceous substrates, such as glass substrates, and plastic substrates, such as polystyrene substrates, are improved with respect to the capability of fixing or immobilization of DNA thereto by treatment with or by providing thereon a coating of an epoxy resin. For example, glass and polystyrene surfaces are improved with respect to the fixing or immobilization of DNA thereon by treatment of glass or polystyrene surfaces with commercially available epoxy glues, such as a solution of epoxy glue in ethanol [1 percent w/v]. These epoxy solutions are applied to the glass, e.g. Pyrex, glass surfaces or polystyrene surfaces or wells, and the solvent, ethanol, evaporated thereupon at a temperature of 37^{o}C, thereby providing a polyamine polymeric coating on the thus-treated surface. These surfaces were found to absorb ³H-labeled DNA from aqueous solution at pH less than 9.5.

The methods described hereinabove with respect to ELISA-like techniques for the colorimetric or photometric determination of the hybridized probes involve enzyme-linked reagents which produce a color change in a substrate or a precipitate. Various combinations of enzymes and color-producing substrates may be employed. See for example accompanying tables, Table I and Table II, showing the combination of enzymes and chromogens. In Table I, the chromogen found in the substrate is reactive with the enzyme employed to elicit or determine the presence of the non-radioactive chemically-labeled probe. The superscript notation (F) indicates that the chromogen fluoresces. In accompanying Table II, there are set forth those chromogens which produce an insoluble product, such as when employed with the avidin/streptavidin system. These combinations of enzyme-chromogen are particularly useful in ELISA techniques for the determination, both qualitatively and quantitatively, of the hybrid DNA or other genetic material containing the special non-radioactive chemically-labeled probe in accordance with this invention.

**TABLE I**

| CHROMOGEN | | |
|---|---|---|
| ENZYME | | |
| 1a. | alkaline phosphatase | 4-Methylumbelliferyl phosphate^{(F)} |
| b. | acid phosphatase | bis (4-Methylumbelliferyl phosphate)^{(F)} 3-0-methylfluorescein. |
| | | Flavone-3-diphosphate triammonium^{(F)} salt p-nitrophenyl phosphate 2Na. |
| 2. | peroxidase | Tyramine hydrochloride^{(F)} |
| | | 3-(p-hydroxyphenyl) Propionic acid^{(F)} |
| | | p-Hydroxyphenethyl alcohol^{(F)} |
| | | 2,2'-Azino-Di-3-Ethylbenzthiazoline sulfonic acid (ABTS) |
| | | ortho-phenylenediamine 2HCl |
| | | o-dianisidine |
| | | 5-aminosalicylic acid |
| | | p-cresol^{(F)} |
| | | 3,3'-dimethyloxybenzidine |
| | | 3-methyl-2-benzothiazoline hydrazone tetramethyl benzidine |
| 3. | β-D-galactosidase | 0-nitrophenyl β-D-galactopyranoside |
| | | 4-methylumbelliferyl-β-D-galactoside |
| 4. | glucose-oxidase | ABTS |

**TABLE II**

| CHROMOGEN (insoluble product) | | |
|---|---|---|
| ENZYME | | |
| 1. | alkaline phosphates | 6-bromo-3-hydroxy-2-naphthyl-0 anisidine + Fast Violet B salt. |
| | | 2,2'-dimethyl-3-hydroxy-2-naphthanilide + Fast Red salt. |
| | | flavone-3-diphosphate ammonium salt + AlCl₃^{(F)} |
| | | 5-bromo-4-chloro-3-indolylphosphate + tetrazolium, nitro BT. |
| 2. | Horseradish peroxidase | H₂O₂ + diaminobenzidine |
| | | H₂O₂ + tetramethylbenzidine |
| 3. | Glucose oxidase | glucose + thiazolyl blue |
| 4. | β-galactosidase | 2-(β-D-galactosidoxy) naphthol AS-LC + Fast Violet B salt. |
| | | Naphthol-AS-Bl-B-0-galactopyranoside + Fast Blue BBN salt. |

## Claims

1. A method for detecting a polynucleotide sequence which comprises:
- fixing said polynucleotide sequence to a solid support which comprises or is contained within a transparent or translucent system, such that the polynucleotide is in a single-strand form and is capable of hybridizing to complementary nucleic acid sequences;
- forming an entity comprising said polynucleotide sequence hybridized to a polynucleotide or oligonucleotide probe, said probe having attached thereto a chemical label comprising a signalling moiety capable of generating a signal; and
- generating and detecting a signal, characterized in that the the transparent or translucent system is a non-porous system and the generated signal is a soluble signal.

2. The method according to claim 1, wherein said detecting step comprises spectrophotometric techniques.

3. The method according to claim 1, wherein said soluble signal is a colored product, a chemiluminescent product or a fluorescent product.

4. The method according to claim 1, wherein said signalling moiety is an enzyme, a chelating agent or a co-enzyme.

5. The method according to claim 1, wherein said solid support is glass, plastic, polystyrene, polyethylene, dextran or polypropylene.

6. The method according to claim 1, wherein said polynucleotide sequence is directly fixed to said solid support.

7. The method according to claim 6, wherein said polynucleotide sequence is fixed to said solid support in single stranded form.

8. The method according to claim 1, wherein said signalling moiety is attached to said polynucleotide or oligonucleotide probe through the formation of a complex.

9. The method according to claim 8, wherein said complex is biotin and avidin, biotin and streptavidin, or a sugar and a lectin.

10. The method according to claim 1, wherein said forming step further comprises washing to remove said polynucleotide or oligonucleotide probes that do not form said entity.

11. The method according to claim 10, wherein said forming step further comprises attaching said signalling moiety to said polynucleotide or oligonucleotide probe after said washing step.

12. The method according to claim 11, which further comprises separating free signalling moieties from said attached signalling moieties.

13. The method according to claim 1, wherein said detecting step further comprises generating said soluble signal in a device capable of transmitting light therethrough for the detection of said soluble signal by spectrophotometric techniques.

14. The method according to claim 13, wherein said device is a well, a tube, a cuvette or an apparatus which comprises a plurality of said wells, tubes or cuvettes.

15. The method according to claim 13, wherein said soluble signal is a colored product, a chemiluminescent product, or a fluorescent product.

16. The method according to claim 13, wherein said solid support and said device are composed of the same materials.

17. A device for detecting a polynucleotide sequence according to the method of claim 1, which device comprises a solid support, having said polynucleotide sequence fixed thereto in hybridized form.

18. A kit for detecting a polynucleotide sequence, which comprises the device of claim 17 in packaged combination with a container of an oligonucleotide or polynucleotide probe, having covalently attached thereto a chemical label comprising a signalling moiety capable Of generating a soluble signal.

19. The kit of claim 18, wherein said soluble signal is a colored product, a chemiluminescent product or a fluorescent product.

20. The method according to claim 1, wherein part of the solid support is modified to facilitate fixing of the polynucleotide sequence to the solid support by treating it with aqueous nitric acid and γ-aminopropyltriethoxysilane.

21. The method according to claim 1, wherein part of the solid support is modified to facilitate fixation of the polynucleotide sequence to the solid support by treating the solid support with a coating of an epoxy resin.

22. The method according to claim 21, wherein part of the solid support is modified to facilitate fixation of the polynucleotide sequence to the solid support by treating the support with the epoxy resin by the following sequential steps:
(a) applying an epoxy glue in solution with ethanol to the solid support; and
(b) evaporating the ethanol by heating to a temperature of about 37°C to provide a polyamine polymeric coating on the solid support.

23. The method according to claim 1, wherein said polynucleotide sequence is fixed to said solid support in double-stranded form, and denatured into single-stranded form prior to the hybridisation step.

24. The method according to claim 1, wherein said polynucleotide sequence in double-stranded form is denatured, and fixed to said solid support in single-stranded form prior to the hybridization step.

25. The method according to claim 1, wherein said signalling moiety is a chemiluminescent agent.

26. A transparent or translucent solid, non-porous substrate having fixed thereto a double-stranded polynucleotide, one of the strands of said double-stranded polynucleotide being a non-radioactive chemically labeled polynucleotide or comprising a non-radioactive chemically-labelled nucleotide as a nucleotide component of said one strand, wherein said chemically labelled polynucleotide comprises or has attached thereto a chemical label comprising a signalling moiety which generates a soluble signal which is detected spectrophotometrically.

27. A substrate in accordance with claim 26, wherein said substrate is an epoxy or epoxy-coated substrate.

28. A substrate in accordance with claim 26, wherein said substrate has amino groups attached or fixed to the surface thereof.

29. A method for detecting a polynucleotide sequence which comprises:
fixing a polynucleotide or oligonucleotide probe, which has attached thereto a chemical label comprising a signalling moiety capable of generating a signal, to a solid support which comprises or is contained within a transparent or translucent system, such that said probe is in single-stranded form and is capable of hybridizing to complementary nucleic acid sequences;
forming an entity comprising said probe hybridized to said polynucleotide sequence, and
generating and detecting a signal, characterized in that the transparent or translucent system is non-porous and the generated signal is a soluble signal.

## Patentansprüche

1. Verfahren zum Nachweis einer Polynucleotidsequenz, umfassend:
- Fixierung der Polynucleotidsequenz an einem festen Träger, der ein durchsichtiges oder lichtdurchlässiges System umfaßt oder in einem solchen enthalten ist, so daß das Polynucleotid in der Einzelstrangform vorliegt und fähig ist, mit komplementären Nucleinsäuresequenzen zu hybridisieren;
- Bildung einer Einheit, umfassend die mit einer Polynucleotid- oder Oligonucleotidsonde hybridisierte Polynucleotidsequenz, wobei an die Sonde ein chemischer Marker geknüpft ist, der eine Signaleinheit umfaßt, die zur Erzeugung eines Signals fähig ist; und
- Erzeugung und Nachweis eines Signals,
dadurch gekennzeichnet, daß das durchsichtige oder lichtdurchlässige System ein nicht-poröses System ist und das erzeugte Signal ein lösliches Signal ist.

2. Verfahren nach Anspruch 1, wobei der Nachweisschritt spektrophotometrische Techniken umfaßt.

3. Verfahren nach Anspruch 1, wobei das lösliche Signal ein gefärbtes Produkt, ein chemilumineszierendes Produkt oder ein fluoreszierendes Produkt ist.

4. Verfahren nach Anspruch 1, wobei die Signaleinheit ein Enzym, ein Chelatmittel oder ein Coenzym ist.

5. Verfahren nach Anspruch 1, wobei der feste Träger Glas, Kunststoff, Polystyrol, Polyethylen, Dextran oder Polypropylen ist.

6. Verfahren nach Anspruch 1, wobei die Polynucleotidsequenz direkt an den festen Träger fixiert ist.

7. Verfahren nach Anspruch 6, wobei die Polynucleotidsequenz in Einzelstrangform an den festen Träger fixiert ist.

8. Verfahren nach Anspruch 1, wobei die Signaleinheit über die Erzeugung eines Komplexes an die Polynucleotid- oder Oligonucleotidsonde geknüpft ist.

9. Verfahren nach Anspruch 8, wobei der Komplex Biotin und Avidin, Biotin und Streptavidin oder ein Zucker und ein Lectin ist.

10. Verfahren nach Anspruch 1, wobei der Bildungsschritt weiterhin einen Waschschritt umfaßt, um die Polynucleotid- oder Oligonucleotidsonden zu entfernen, die diese Einheit nicht bilden.

11. Verfahren nach Anspruch 10, wobei der Bildungsschritt weiterhin das Anknüpfen der Signaleinheit an die Polynucleotid- oder Oligonucleotidsonde nach dem Waschschritt umfaßt.

12. Verfahren nach Anspruch 11, weiterhin umfassend die Abtrennung der freien Signaleinheiten von den verknüpften Signaleinheiten.

13. Verfahren nach Anspruch 1, wobei der Nachweisschritt weiterhin die Erzeugung des löslichen Signals in einer Vorrichtung umfaßt, die fähig ist, Licht für den Nachweis des löslichen Signals durch spektrophotometrische Techniken hindurchzulassen.

14. Verfahren nach Anspruch 13, wobei die Vorrichtung eine Vertiefung, ein Röhrchen, eine Küvette oder eine Anordnung ist, die eine Vielzahl solcher Vertiefungen, Röhrchen oder Küvetten umfaßt.

15. Verfahren nach Anspruch 13, wobei das lösliche Signal ein gefärbtes Produkt, ein chemilumineszierendes Produkt oder ein fluoreszierendes Produkt ist.

16. Verfahren nach Anspruch 13, wobei der feste Träger und die Vorrichtung aus dem gleichen Material bestehen.

17. Vorrichtung zum Nachweis einer Polynucleotidsequenz gemäß dem Verfahren nach Anspruch 1, wobei die Vorrichtung einen festen Träger umfaßt, der die in hybridisierter Form daran fixierte Polynucleotidsequenz umfaßt.

18. Kit zum Nachweis einer Polynucleotidsequenz, umfassend die Vorrichtung nach Anspruch 17 abgepackt in Kombination mit einem Behälter einer Oligonucleotid- oder Polynucleotidsonde, mit einem daran kovalent geknüpften chemischen Marker, der eine Signaleinheit, die zur Erzeugung eines löslichen Signals fähig ist, umfaßt.

19. Kit nach Anspruch 18, wobei das lösliche Signal ein gefärbtes Produkt, ein chemilumineszierendes Produkt oder ein fluoreszierendes Produkt ist.

20. Verfahren nach Anspruch 1, wobei ein Teil des festen Trägers durch Behandlung mit wäßriger Salpetersäure und γ-Aminopropyltriethoxysilan modifiziert wird, um die Fixierung der Polynucleotidsequenz an den festen Träger zu erleichtern.

21. Verfahren nach Anspruch 1, wobei ein Teil des festen Trägers durch Behandlung des festen Trägers mit einem Film eines Epoxyharzes modifiziert wird, um die Fixierung der Polynucleotidsequenz an den festen Träger zu erleichtern.

22. Verfahren nach Anspruch 21, wobei ein Teil des festen Trägers zur Erleichterung der Fixierung der Polynucleotidsequenz an den festen Träger modifiziert wird, wobei der Träger mit Epoxyharz gemäß den folgenden Schritten behandelt wird:
(a) Aufbringen eines Epoxyklebstoffes in Lösung mit Ethanol auf den festen Träger; und
(b) Abdampfen des Ethanols durch Erwärmen auf eine Temperatur von etwa 37°C, um einen Polyamin-Polymerfilm auf dem festen Träger bereitzustellen.

23. Verfahren nach Anspruch 1, wobei die Polynucleotidsequenz in Doppelstrangform an den festen Träger fixiert ist und vor dem Hybridisierungsschritt in die Einzelstrangform denaturiert wird.

24. Verfahren nach Anspruch 1, wobei die Polynucleotidsequenz in Doppelstrangform denaturiert wird und in Einzelstrangform vor dem Hybridisierungsschritt an den festen Träger fixiert wird.

25. Verfahren nach Anspruch 1, wobei die Signaleinheit ein chemilumineszierendes Mittel ist.

26. Durchsichtiges oder lichtdurchlässiges festes, nicht poröses Substrat, an das ein doppelsträngiges Polynucleotid fixiert ist, wobei einer der Stränge des doppelsträngigen Polynucleotids ein nicht radioaktives chemisch markiertes Polynucleotid ist, oder ein nicht radioaktives chemisch markiertes Nucleotid als Nucleotidkomponente des einen Stranges umfaßt, wobei das chemisch markierte Polynucleotid einen chemischen Marker umfaßt oder daran geknüpft hat, der eine Signaleinheit umfaßt, die ein spektrophotometrisch nachgewiesenes lösliches Signal erzeugt.

27. Substrat nach Anspruch 26, wobei das Substrat ein Epoxy- oder Epoxy-überzogenes Substrat ist.

28. Substrat nach Anspruch 26, wobei das Substrat Aminogruppen an die Oberfläche geknüpft oder fixiert hat.

29. Verfahren zum Nachweis einer Polynucleotidsequenz, umfassend:
Fixierung einer Polynucleotid- oder Oligonucleotidsonde an einen festen Träger, der ein durchsichtiges oder lichtdurchlässiges System umfaßt oder in einem solchen enthalten ist, wobei an die Sonde ein chemischer Marker geknüpft ist, der eine Signaleinheit umfaßt, die fähig ist, ein Signal zu erzeugen, so daß die Sonde in Einzelstrangform vorliegt und fähig ist, mit komplementären Nucleinsäuresequenzen zu hybridisieren;
Bildung einer Einheit, umfassend die mit der Polynucleotidsequenz hybridisierte Sonde, und
Erzeugung und Nachweis eines Signals, dadurch gekennzeichnet, daS das durchsichtige oder lichtdurchlässige System nicht porös ist und das erzeugte Signal ein lösliches Signal ist.

## Revendications

1. Procédé pour détecter une séquence de polynucléotide qui comprend :
- la fixation de cette séquence de polynucléotide à un support solide qui comprend ou est contenu dans un système transparent ou translucide, de telle sorte que le polynucléotide soit sous la forme à simple brin et puisse s'hybrider à des séquences d'acides nucléiques complémentaires;
- la formation d'une entité comprenant cette séquence de polynucléotide hybridée à une sonde de polynucléotide ou d'oligonucléotide, sonde à laquelle est attachée un traceur chimique comprenant un fragment de signalisation pouvant produire un signal; et
- la production et la détection d'un signal,
caractérisé en ce que le système transparent ou translucide est un système non poreux et le signal produit est un signal soluble.

2. Procédé suivant la revendication 1, caractérisé en ce que l'étape de détection comprend des techniques spectrophotométriques.

3. Procédé suivant la revendication 1, caractérisé en ce que le signal soluble est un produit coloré, un produit chimioluminescent ou un produit fluorescent.

4. Procédé suivant la revendication 1, caractérisé en ce que le fragment de signalisation est une enzyme, un agent de chélation ou une coenzyme.

5. Procédé suivant la revendication 1, caractérisé en ce que le support solide est du verre, une matière plastique, du polystyrène, du polyéthylène, du dextrane ou du polypropylène.

6. Procédé suivant la revendication 1, caractérisé en ce que la séquence de polynucléotide est directement fixée au support solide.

7. Procédé suivant la revendication 6, caractérisé en ce que la séquence de polynucléotide est fixée au support solide sous la forme à simple brin.

8. Procédé suivant la revendication 1, caractérisé en ce que le fragment de signalisation est fixé à la sonde de polynucléotide ou d'oligonucléotide par l'intermédiaire de la formation d'un complexe.

9. Procédé suivant la revendication 8, caractérisé en ce que le complexe est de la biotine et de l'avidine, de la biotine et de la streptavidine ou un sucre et une lectine.

10. Procédé suivant la revendication 1, caractérisé en ce que l'étape de formation précitée comprend en outre un lavage pour séparer les sondes de polynucléotide ou d'oligonucléotide qui ne forment pas l'entité susdite.

11. Procédé suivant la revendication 10, caractérisé en ce que l'étape de formation comprend en outre la fixation du fragment de signalisation à la sonde de polynucléotide ou d'oligonucléotide après ladite étape de lavage.

12. Procédé suivant la revendication 11, caractérisé en ce qu'elle comprend en outre la séparation des fragments de signalisation libres des fragments de signalisation fixés.

13. Procédé suivant la revendication 1, caractérisé en ce que l'étape de détection précitée comprend en outre la production du signal soluble dans un dispositif pouvant transmettre la lumière à travers celui-ci pour la détection de ce signal soluble par des techniques spectrophotométriques.

14. Procédé suivant la revendication 13, caractérisé en ce que ce dispositif est un puits, un tube, une cuvette ou un appareil qui comprend une pluralité de ces puits, tubes ou cuvettes.

15. Procédé suivant la revendication 13, caractérisé en ce que le signal soluble est un produit coloré, un produit chimioluminescent ou un produit fluorescent.

16. Procédé suivant la revendication 13, caractérisé en ce que le support solide et le dispositif précités sont formés des mêmes matières.

17. Dispositif pour détecter une séquence de polynucléotide suivant le procédé de la revendication 1, lequel dispositif comprend un support solide, auquel la séquence de polynucléotide est fixée sous forme hybridée.

18. Trousse pour détecter une séquence de polynucléotide, qui comprend le dispositif de la revendication 17 en combinaison conditionnée avec un récipient d'une sonde d'oligonucléotide ou de polynucléotide, à laquelle est attaché par covalence un traceur chimique comprenant un fragment de signalisation pouvant produire un signal soluble.

19. Trousse suivant la revendication 18, caractérisée en ce que le signal soluble est un produit coloré, un produit chimioluminescent ou un produit fluorescent.

20. Procédé suivant la revendication 1, caractérisé en ce qu'une partie du support solide est modifiée pour faciliter la fixation de la séquence de polynucléotide au support solide par traitement de celui-ci avec de l'acide nitrique aqueux et du γ-aminopropyltriéthoxysilane.

21. Procédé suivant la revendication 1, caractérisé en ce qu'une partie du support solide est modifiée pour faciliter la fixation de la séquence de polynucléotide au support solide par traitement du support solide avec un revêtement d'une résine époxy.

22. Procédé suivant la revendication 21, caractérisé en ce qu'une partie du support solide est modifiée pour faciliter la fixation de la séquence de polynucléotide au support solide par traitement du support avec la résine époxy par les étapes successives suivantes :
(a) l'application d'une colle époxy en solution avec de l'éthanol sur le support solide; et
(b) l'évaporation de l'éthanol par chauffage à une température d'environ 37°C pour former un revêtement polymère de polyamine sur le support solide.

23. Procédé suivant la revendication 1, caractérisé en ce que la séquence de polynucléotide est fixée au support solide sous la forme à double brin, et dénaturée sous la forme à simple brin avant l'étape d'hybridation.

24. Procédé suivant la revendication 1, caractérisé en ce que la séquence de polynucléotide sous la forme à double brin est dénaturée, et fixée au support solide sous la forme à simple brin avant l'étape d'hybridation.

25. Procédé suivant la revendication 1, caractérisé en ce que le fragment de signalisation est un agent chimioluminescent.

26. Substrat non poreux, solide transparent ou translucide auquel est fixé un polynucléotide à double brin, un des brins de ce polynucléotide à double brin étant un polynucléotide marqué chimiquement non radioactif ou comprenant un nucléotide marqué chimiquement non radioactif comme composant nucléotidique de ce brin, dans lequel le polynucléotide marqué chimiquement comprend ou comporte, attaché à celui-ci, un traceur chimique comprenant un fragment de signalisation qui produit un signal soluble qui est détecté spectrophotométriquement.

27. Substrat suivant la revendication 26, caractérisé en ce que ledit substrat est un substrat d'époxy ou revêtu d'époxy.

28. Substrat suivant la revendication 26, caractérisé en ce que ledit substrat comporte des groupes amino attachés ou fixés à sa surface.

29. Procédé de détection d'une séquence de polynucléotide qui comprend :
la fixation d'une sonde de polynucléotide ou d'oligonucléotide, à laquelle est attaché un traceur chimique comprenant un fragment de signalisation pouvant produire un signal, à un support solide qui comprend ou est contenu dans un système transparent ou translucide, de telle sorte que la sonde soit sous la forme à simple brin et puisse s'hybrider à des séquences d'acides nucléiques complémentaires;
la formation d'une entité comprenant ladite sonde hybridée à ladite séquence de polynucléotide; et
la production et la détection d'un signal, caractérisé en ce que le système transparent ou translucide est non poreux et le signal produit est un signal soluble.
